# EUROPÄISCHE PATENTSCHRIFT

(11) **EP 0 998 912 B1**
(45) Veröffentlichungstag und Bekanntmachung des Hinweises auf die Patenterteilung: **20.10.2004**
(21) Anmeldenummer: 98120762.4
(22) Anmeldetag: 02.11.1998
(51) Int. Cl.: A61K 7/48, C11D 3/39, C11D 3/48

(54) **Verwendung von Persäuren zur Händedesinfektion oder -dekontamination**
Use of peracids for hand's disinfection or decontamination
Utilisation de peracides pour la désinfection ou la décontamination des mains

(43) Veröffentlichungstag der Anmeldung: 10.05.2000
(73) Patentinhaber: Bode Chemie GmbH & Co., 22525 Hamburg (DE)
(72) Erfinder: Rotter, Manfred, Dr. Prof., 1080 Wien (AT); Pietsch, Hanns, Dr. Dipl.-Chem., 20148 Hamburg (DE); Heeg, Peter, Dr. Prof., 72119 Ammerbuch (DE); Brauel, Anke, Dr. Dipl.-Chem., 21077 Hamburg-Harburg (DE); Ostermeyer, Christiane, 22559 Hamburg (DE)
(74) Vertreter: Schaeffer, Michael

(56) Entgegenhaltungen:
- EP-A- 0 469 983
- WO-A-94/24863
- WO-A-98/33490
- DE-A- 3 133 425
- DE-A- 3 615 787
- DE-A- 19 508 827
- GB-A- 2 137 882
- DIN EN 1500
- ZBL. BAKT. HYG. Bd. B182, 1986, Seiten 562 - 570

## Beschreibung

Die Erfindung betrifft den in den Ansprüchen angegebenen Gegenstand.

Der wichtigste Übertragungsweg von Krankheitserregern aller Art sind die Hände; ca. 80 % aller Infektionen werden auf diesem Wege übertragen. Die Dekontamination und/oder Desinfektion der Hände ist somit die wichtigste Maßnahme zur Verhinderung von Infektionen, in dem diese Übertragungskette unterbrochen wird. Die Hautantiseptik hat hingegen eine andere Zielsetzung, denn sie soll bei invasiven Maßnahmen wie Punktion, Inzision oder chirurgischen Eingriffen den Patienten davor schützen, daß durch die perforierte oder eröffnete Haut Keime in den Körper gelangen. Ziel der Händedesinfektion wie auch der Hautantiseptik ist eine weitgehende Keimreduktion, wobei unter den Begriff Keime sowohl Bakterien wie auch Pilze und Viren fallen. Händedekontamination und Händedesinfektion wird überwiegend von Personal vorgenommen, das von berufswegen diese Maßnahmen tagtäglich und in der Regel mehrfach täglich bis zu einem Dutzend mal oder mehr vornehmen muß wie beispielsweise in Krankenhäusern, Arztpraxen, Altenheimen, Unfallstationen, aber auch in Betrieben, die mit der medizinischen Hilfeleistung nichts zu tun haben wie beispielsweise in Nahrungsmittel herstellenden Betrieben, in der Pharmabranche und in weiteren Branchen, in denen unter Reinstraumbedingungen gearbeitet wird. In allen diesen Fällen sind die Hände des Personals über die Dauer der Arbeitszeit gesehen relativ sehr lange mit den Desinfektionsmitteln in Kontakt. Neben der Keimreduktion wird daher von Desinfektions- bzw. Dekontaminationsmittel eine gute Hautverträglichkeit auch bei häufigen Gebrauch gefordert. Bei der Hautantiseptik wird das Präparat an einer Person nur gelegentlich verwendet, aber kritisch in diesen Fällen ist, daß über die Perforierung bzw. Inzision die Wirkstoffe in den Körper eindringen und unerwünschte systemische Effekte verursachen können. Aus diesem Grunde werden an Hautdesinfektionsmittel hohe Anforderungen hinsichtlich der Verträglichkeit und der Nichttoxizisität gestellt, so daß viele germizide Substanzen, die für die Flächen- oder Wäschedesinfektion einsetzbar sind, für die Hautdesinfektion nicht in Frage kommen. Viele Hände- und Hautdesinfektionsmittel basieren auf den Alkoholen Ethanol und/oder 1-Propanol und/oder 2-Propanol. Diese alkoholischen Hautdesinfektionsmittel, die in der Regel etwa 60 bis 95 Gewichts-% Alkohol ggf. mit weiteren Wirkstoffen oder Farb-, Duft- und Hautpflegestoffen enthalten sind, sind schnell innerhalb von 30 bis 60 Sek. gegen eine breite Palette von Keimen wirksam. Daneben werden zur Händedekontamination vielfach auch tensidhaltige Produkte eingesetzt, die bei der Anwendung ähnlich wie Seifen benutzt werden. Die für diese Zubereitungen üblicherweise verwandten antimikrobiellen Wirkstoffe sind in der Regel Jod, PVP-Jod, Chlorhexidin oder Triclosan. Händedesinfektion und Händedekontamination verlangen beide Reinigung und Keimzahlreduktion, jedoch werden bei letzterer bei der Dekontamination etwas geringere Anforderungen als bei der chirurgischen Händedesinfektion gestellt. Die Anforderungen hinsichtlich der Wirksamkeit sind z.B. in Deutschland festgelegt in den Prüfmethoden der DGHM und werden für Europa demnächst in den Normen des CEN, die jetzt bereits als Normentwürie vorliegen, festgelegt werden.

Die bisher genannten Wirkstoffe und Wirkstoffkombinationen sind hinsichtlich ihrer Wirksamkeit gegen Bakterien als auch gegen Pilze und Viren bekannt und seit Jahren bewährt.

Andererseits werden die Anforderungen an die Hautverträglichkeit immer höher. Häufiges Waschen mit diesen Wirkstoffen ist eine starke Noxe, die zu dauernden Hautschäden führen kann. Dabei wird der Haupteffekt durch das Herauswaschen der Hautfette verursacht, insbesondere wenn die Präparate Tenside oder Alkohole enthalten. Die übermäßige Entfettung der Haut führt zur Rissigkeit bis hin zum Krankheitsbild der Chrirurgenhände.

Auch die weiteren heute üblichen Wirkstoffe weisen hinsichtlich ihrer Verträglich beträchtliche Defizite auf. Jod und PVP-Jod verursachen bei Überempfindlichkeit häufig Allergien, außerdem wird die Haut stark verfärbt und Jod penetriert, insbesondere in alkoholischen Lösungen durch die Haut, was bei empfindlichen Personen zur Hyperthyreose und auch zum Jodismus führen kann. Bei PVP-Jod sind diese Nebenwirkungen zwar geringer, jedoch ebenfalls manifest. Chlorhexidin und seine Salze sind weltweit neben Jod und PVP-Jod sowie den Alkoholen der wichtigste Wirkstoff für die Hautdesinfektion und Wundantiseptik, obwohl diese Verbindungen in toxikologischer Hinsicht kritisch beurteilt werden. Chlorhexidin ist im Ames-Test und im DNA-repair-Test positiv. Beide Ergebnisse deuten auf mutagenes Potential hin. Die Abbauprodukte p-Chloranilin und p-Chlorphenylisocyanat haben eine große Affinität zur Haut und kumulieren bei häufigem Gebrauch. Triclosan, ein chloriertes Phenol, penetriert sehr stark durch die Haut und ist ein potentieller Dioxinbildner.

Weiterhin ist zu beachten, daß die weltweit am häufigsten verwendeten Wirkstoffe, wie Jod und andere Halogene, Chlorhexidin und Diphenole eine beträchtliche Belastung der Umwelt darstellen, wenn sie wie in Krankenhäusern oder Hospitälern in größeren Mengen in das Abwasser oder in die Umgebungsluft gelangen.

Außerdem lehrt die Erfahrung, daß zwar beim fachkundigen Personal die Regeln der chirurgischen Händedesinfektion, also die Beseitigung der transienten und der residenten Flora durch Bürsten mit desinfizierenden Lösungen, Nagelreinigung und ein 3- bis 5-minütiges Wiederholen des Einreibens der Hände mit einem wirksamen Desinfektionsmittel, in der Regel eingehalten werden, das aber selbst ausgebildete Mediziner dazu neigen, die als lästige Pflicht empfundene Händedekontamination in möglichst kurzer Zeit hinter sich zu bringen und daher häufig die notwendige Einwirkzeit der Desinfektionsmittel nicht eingehalten wird.

Es besteht daher noch ein Bedarf nach einem Mittel zur Dekontamination und Desinfektion der Haut und insbesondere der Hände, das schnell, d.h. also innerhalb von 30 bis höchstens 60 Sekunden wirkt, die Haut nicht entfettet, keine Allergien oder toxischen Nebenwirkungen verursacht und nicht umweltbelastend ist.

Erfindungsgemäß wird jetzt die Verwendung von physiologisch verträglichen Persäuren oder deren Salzen zur hygienischen und chirurgischen Händedesinfektion in Mengen von 0,1 bis 10 Gewichts-% in rein wässriger Lösung, mit einer Keimreduktion gemäß den Richtlinien der DGHM in 30 bis 60 sekunden, entsprechend den Anforderungen im ein Desinfektionsmittel mit 60 Vol.% Ethanol oder Isopropanol, wobei als Persäure Magnesiummonoperoxiphthalat eingesetzt wird, vorgeschlagen.

Die germizide Wirkung von Persäuren, die auf der Bildung von Wasserstoffperoxid beruht ist an und für sich bekannt. Zur Desinfektion sind daher schon vorgeschlagen worden diverse aliphatische Persäuren, wie Peressigsäure oder Perpropionsäure oder aromatische Persäuren wie Perbenzoesäure oder Monoperophthalsäure. Wegen der leichten Zugänglichkeit und einer hinreichenden Stabilität ist insbesondere Magnesium-Monoperoxidophthalat als Wasch- und Bleichmittel oder als Desinfektionsmittel vorgeschlagen worden. Der Einsatz dieser Verbindung, im folgenden als MMPP abgekürzt, als Wasch- und Bleichmittel ergibt sich beispielsweise aus der EP 0 027 693 und EP 0 024 367; die Verwendung als Flächendesinfektionsmittel wird u.a. in der GB 2 357 630 oder GB 2 137 882 beschrieben. In der PCT WO 87/01562 wird MMPA zum Reinigen und Desinfizieren von Kontaktlinsen vorgeschlagen, in der US 4,990,329 wird die Substanz für desinfizierende Mundspülungen beschrieben.

DE 195 08 827 A1 beschreibt Wund- und Schleimhautantiseptika auf der Basis von Magnesiummonoperphthalat als trockenes Granulat, Tabletten oder Brausetabletten, welche(s) vor Gebrauch in Wasser aufgelöst und als wässerige Lösung verwendet (wird) werden.

DE 36 15 787 A1 beschreibt Flächendesinfektionsmittel, bestehend aus Sauerstoffdonatoren (Magnesiummonoperphthalat) und Acyldonatoren wie Tetraacetylethylendiamin, Pentaacetylglukose oder Tetraacetylglykoluril. Wirksames Agens ist die Peressigsäure, welche beim Auflösen der Zubereitung in Wasser entsteht.

DE 31 33 425 A1 beschreibt topische, kosmetische Zusammensetzungen mit einem Gehalt von 0,1 bis 20 Gew.-% einer Percarbonsäure zur Verbesserung des fettigen und unästhetischen Aussehens der Haare und der Haut, d. h. es wird eine sebosuppressive Wirksamkeit beschrieben. Die Zubereitungen können Lösungen, Suspensionen, Gele, Emulsionen, Salben, Pasten und Aerosole sein.

WO 98/33490 beschreibt Mittel gegen haarige Warzen von Milchvieh (Papillomatus Digital Dermatitis) verursacht durch Spirochäten wie zum Beispiel Fusobacterium mecrophorum, Dichelobacter nodosus, Bacteroides melaninogenicus. Das beschriebene Mittel enthält eine verdünnte Percarbonsäure, bevorzugt Peressigsäure.

Aus dem Stand der Technik ist bekannt, daß MMPP eine gute mikrobiozide Wirksamkeit und auch eine gute Haut- und Schleimhautverträglichkeit hat. Bis heute hin ist man aber davon ausgegangen, daß MMPP wie auch die anderen Persäuren und ihre Salze bei Umgebungstemperatur nur langsam wirkt, so daß bereits zahlreiche Vorschläge gemacht worden sind, diese Wirkung durch Temperaturerhöhung oder durch Zugabe von Aktivatoren der verschiedensten Art zu verbessern; so beschreibt die GB 2 257 630 die Verwendung von Eisen III- oder Kupfersalz, die EP 0 024 367 die Zugabe von Bromid, die DE 3 240 565 die Zugabe von Chelatbildnern und die DE 3 615 787 die Verwendung von mindestens drei organischen Aktivatoren, wobei diese Aktivatoren der verschiedensten Art häufig in großen Mengen bis zu 50 Gewichts-% zugesetzt werden. Die Zugabe von organischen Aktivatoren, von Schwermetall- oder Eisensalz oder von Bromid ist aber keineswegs wünschenswert, da hierdurch einerseits Allergien bei entsprechend disponierten Personen ausgelöst werden können und andererseits derartige Verbindungen selbst wieder zu einer nicht unbedeutenden Umweltbelastung führen können.

Völlig überraschend wurde jetzt festgestellt, daß die bisherige Annahme, MMPP wäre als Desinfektionsmittel bei Umgebungstemperatur zu langsam", überhaupt nicht zutrifft. Vielmehr hat sich jetzt herausgestellt, daß 1 bis 10 gewichts-%-ige rein wässrige Lösungen von MMPA eine vergleichbare Keimreduktion bei der hygienischen Händedesinfektion gemäß den Richtlinien der DGHM bzw. den Euronorm-Entwürfen in 30 bis 60 Sekunden entsprechend den Anforderungen an ein Desinfektionsmittel mit 60 Volumen-% Ethanol oder Isopropanol ergeben. Diese schnelle Desinfektion mit einem vergleichbaren Zeitfaktor wie die sofort wirkenden Alkohole war aufgrund der bisherigen Aussagen in keiner Weise zu erwarten. Vergleichsversuche haben ergeben, daß 0,1 bis 10 gewichts-%-ige wässrige Lösungen von MMPP nicht nur hervorragend hautverträglich sind, sondern in diesen Konzentrationen antibakterielle, antimykotische und antivirale Eigenschaften, letztere insbesondere gegen unbehüllte Viren, aufweisen, d.h. also, daß wässrige Lösungen von MMPP in diesen Konzentrationen in einem Zeitraum von etwa 30 Sekunden Einwirkzeit bei Bakterien eine Keimreduktion von über 4 log-Stufen entsprechend 99,99 % aufweisen.

MMPP kann als Schnelldekontaminations- bzw. -desinfektionsmittel in einfacher Weise als kristallines Pulver in vordosierter Form oder als Granulat, Perlen, Tabletten oder Brausetabletten in einer vorgegebenen Wassermenge aufgelöst werden, mit der die Hände eingerieben werden. Die Herstellung entsprechender Granulate, Perlen, Tabletten oder Brausetabletten ist dem Fachmann geläufig und braucht nicht im einzelnen beschrieben zu werden. Pulver und Granulate sollten, um eine Staubentwicklung zu vermeiden, vorzugsweise eine Partikelgräße von über 100 µm aufweisen.

Des erfindungsgemäß eingesetzte MMPP Kann aber auch als Pasten oder pumpfähige Dispersionen hergestellt werden, wobei allerdings als Träger hydmphile, wasserlösliche, aber wasserfreie Verbindungen eingesetzt werden müssen, um eine entsprechende Stabilität zu gewährleisten. Als Zubereitung in einem wasserfreien Träger sind die Verbindungen in der Regel über ein Jahr stabil. Als Träger eignen sich physiologisch gut verträgliche Glycerin- oder Glykolester oder -ether wie Glykodiacetat oder Glykoldimethyl- bzw. -diethylether oder Glycerintriacetat bzw. die -trimethyl- oder -triethylether oder niedere Polyethylenoxide mit einem Molekulargewicht von etwa 1.000 bis 1.500, die Flüssigkeiten darstellen. Derartige Pasten bzw. Dispersionen können durch Pumpspender proportioniert oder in vordosierter Form angebogen werden, so daß sie nur in einer vorgegebenen Menge Wasser aufgelöst werden müssen. Derartige Pasten oder Dispersionen enthalten etwa 10 bis 50 % MMPP, ggf. auf Wunsch mit einem Zusatz von wasserfreien Tensiden wie Natriumlaurylsulfat.

Die wässrigen Lösungen der erfindurgsgemäßen Schnelldekontaminationoder -desinfektionsmittel, gleichgültig, ob sie aus Pulver, festen Zubereitungsformen oder Dispersionen in wasserfreien Trägern hergestellt worden sind, sind bei Raumtemperatur ungefähr 24 Stunden haltbar, so daß sie bei entsprechenden Notwendigkeiten wie beispielsweise Reihenuntersuchungen, Massenunfällen usw. auch in größerer Menge vorsorglich bereitgestellt werden können. Falls zusätzlich eine reinigende Wirkung erwünscht ist, können die erfindungsgemäßen Zubereitungen sowohl in trockner sowie auch in flüssiger Form zusätzlich auch Tenside als Hilfsstoffe enthalten. Vorzugsweise werden als geeignete Tenside Natriumalkylsulfate bzw. -sulfonate eingesetzt, da diese die Wirksamkeit nicht beeinträchtigen.

Die Erfindung wird nunmehr im folgenden anhand der Beispiele näher erläutert:

### Beispiel 1:

### Chirurgische Händedesinfektion

Aus granuliertem kristallinen MMPP-Hexahydrat wurde durch Auflösung demineralisiertem Wasser eine 5 gew.-%ige Lösung hergestellt.

Mit dieser Lösung wurde der Test zur chirurgischen Händedesinfektion entsprechend den Richtlinien der DGHM (Stand: 01.01.1981, Versuch II-2) durchgeführt. Bei diesem Versuch werden die Hände durch Einreiben 3 Minuten feucht gehalten und gegen ein Standardalkohol (n-Propanol 60 Vol.-%) nach 5 Minuten getestet. Der Test gilt als bestanden, wenn die residente Hautflora durch das Testpräparat gleich oder stärker reduziert wird als durch den Standard.

Eine 5 gew.-%ige MMPP-Lösung erfüllt diese Anforderungen und ist somit für die chirurgische Händedesinfektion geeignet. Es wurden keine Hautunverträglichkeiten festgestellt.

### Beispiel 2:

### Händedekontamination

Aus 80,0 Gew.-% MMPP-Hexahydrat, 10,0 % Natriumcumolsulfonat, 0,8 % Methylhydroxycellulose, 7,6 % Alkylbenzolsulfonat und 1,6 % eines ethoxylierten Fettalkohol wird ein Granulat hergestellt. Von diesem Granulat wird durch Lösen in Leitungswasser eine 5 gew.-%ige wässrige Lösung erzeugt.

Mit einer solchen Lösung, die 4 Gew.-% des Wirkstoffes MMPP enthält, wurde ein Test zur Händedekontamination nach DGHM (Stand: 08.07.1986, Versuch 11) durchgeführt. Bei diesem Verfahren wird die Fähigkeit getestet, die transiente Hautflora zu reduzieren. Der Test gilt als bestanden, wenn das Präparat innerhalb von 30 Sekunden eine E. coli-Kontamination um 3,5 logarithmische Stufen entsprechend 99,9684 % reduziert.

Die Lösung entsprach den Anforderungen des Tests. Hinweise auf Hautirritationen wurden nicht gefunden.

### Beispiel 3:

### Händedekontamination

Es wurden Suspensionen bzw. Pasten mit folgender Zusammensetzung hergestellt:
a) 60 % Diethylsuccinat
   34 % MMPP
   5 % Natriumlaurylsulfonat
   1 % Tetradecanol
b) 60 % Glycoldiacetat
   34 % MMPP
   5 % Natriumlaurylsulfonat
   1 % Tetradecanol
c) 60 % Ethylacetat
   34 % MMPP
   5 % Natriumlaurylsulfonat
   1 % Tetradecanol
d) 60 % Glycerintriacetat
   34 % MMPP
   5 % Natriumlaurylsulfat

Mit diesen Pasten wurden entsprechend Beispiel 2 die Versuche zur Händedekontamination durchgeführt. Die Hände werden 30 Sekunden mit 1,5 ml der Pasten eingerieben und dann mit Wasser entsprechend der Vorschrift aufgeschäumt.

Ergebnis der Versuche war, daß die transiente Flora (E. coli) in 30 Sekunden um 3,5 log-Stufen reduziert wurde. Hautreizungen wurden nicht festgestellt.

### Beispiel 4:

### Hygienische Händedesinfektion

Mit der Zubereitung aus Beispiel 2 wurde der Test zur hygienischen Händedesinfektion gemäß den Richtlinien der DGHM (Stand: 01.01.1981, Versuch II-1) durchgeführt. Bei diesem Versuch muß die transiente Flora (E. coli) in 30 Sekunden gleich oder stärker reduziert werden als mit einem Standardalkohol (Isopropanol 60 Vol.-%) in 1 Minute.

Bei dem Versuch wurde eine ausreichende Reduktion in 30 Sekunden erzielt, wobei keine Hautreizungen festzustellen waren.

## Patentansprüche

1. Verwendung von physiologisch verträglichen Persäuren oder deren Salzen zur hygienischen und chirurgischen Hände-desinfektion oder -dekontamination mit einer Keimreduktion gemäß den Richtlinien der DGHM in 30 bis 60 Sekunden, entsprechend den Anforderungen an ein Desinfektionsmittel mit 60 Vol.-% Ethanol oder Isopropanol, mit einem Gehalt von 0,1 bis 10 Gewichts-% Magnesium-monoperoxyphthalat in wässeriger Lösung.

2. Verwendung nach Anspruch 1, **dadurch gekennzeichnet, dass** das Mittel in fester, zur Auflösung in Wasser bestimmter Form als Pulver, Granulat, Perlen, Tabletten oder Brausetabletten vorliegt.

3. Verwendung nach Anspruch 1 bis 2, **dadurch gekennzeichnet, dass** das Mittel als Pulver oder Granulat mit einer Partikelgröße von über 100 µm vorliegt.

4. Verwendung nach Anspruch 1, **dadurch gekennzeichnet, dass** das Mittel als Paste oder pumpfähige Dispersion in einem hydrophilen wasserlöslichen Träger vorliegt.

5. Verwendung nach Anspruch 1 und 4, **dadurch gekennzeichnet, dass** als Träger Glycoldimethylether, Glycoldiethylether, Glyzerintrimethylether, Glycerintriethylether, Glycoldiacetat, Glycerintriacetat und/oder Polyethylenglycole mit einem Molekulargewicht zwischen 1.000 bis 1.500 enthalten sind.

6. Verwendung nach Anspruch 1 bis 5, dadurch gekemzeichnet, dass das Mittel zusätzlich Tenside, insbesondere Alkylsulfate oder -sulfonate enthält.

## Claims

1. Use of physiologically acceptable per-acids or salts thereof for hygienic and surgical disinfection or decontamination of hands with a germ reduction in accordance with DGHM [German Association of Hygiene and Microbiology] guidelines in 30 to 60 seconds, corresponding to the requirements of a disinfectant with 60 vol% ethanol or isopropanol, having a content of 0.1 to 10 % by weight of magnesium monoperoxyphthalate in aqueous solution.

2. Use according to claim 1, **characterized in that** the composition is in a solid form, intended for dissolution in water, as a powder, granules, beads, tablets or effervescent tablets.

3. Use according to claim 1 to 2, **characterized in that** the composition is in the form of a powder or granules having a particle size of more than 100 µm.

4. Use according to claim 1, **characterized in that** the composition is in the form of a paste or pumpable dispersion in a hydrophilic water-soluble carrier.

5. Use according to claim 1 and 4, **characterized in that** the composition comprises, as the carrier, glycol dimethyl ether, glycol diethyl ether, glycerol trimethyl ether, glycerol triethyl ether, glycol diacetate, glycerol triacetate and/or polyethylene glycols having a molecular weight of between 1,000 and 1,500.

6. Use according to claim 1 to 5, **characterized in that** the composition additionally comprises surfactants, in particular alkylsulphates or -sulphonates.

## Revendications

1. Emploi de peracides physiologiquement admissibles, ou de sels de tels peracides, pour la désinfection ou la décontamination des mains, pour hygiène ou en chirurgie, avec, en 30 à 60 secondes, réduction des germes selon les directives de la DGHM, qui correspond à ce qu'on exige d'un désinfectant à 60 % en volume d'éthanol ou d'isopropanol, avec du mono-peroxyphtalate de magnésium en solution aqueuse en une concentration de 0,1 à 10 % en poids.

2. Emploi conforme à la revendication 1, **caractérisé en ce que** l'agent se présente sous une forme solide adaptée pour dissolution dans de l'eau, telle que poudre, granulés, perles, comprimés ou comprimés effervescents.

3. Emploi conforme à la revendication 1 ou 2, **caractérisé en ce que** l'agent se présente sous forme de poudre ou de granulés en particules de taille supérieure à 100 µm.

4. Emploi conforme à la revendication 1, **caractérisé en ce que** l'agent se présente sous forme de pâte ou de dispersion pompable dans un véhicule hydrophile hydrosoluble.

5. Emploi conforme aux revendications 1 et 4, **caractérisé en ce que** le véhicule contient de l'éther diméthylique de glycol, de l'éther diéthylique de glycol, de l'éther triméthylique de glycérol, de l'éther triéthylique de glycérol, du diacétate de glycol, du triacétate de glycérol et/ou des polyéthylèneglycols dont la masse moléculaire vaut de 1000 à 1500.

6. Emploi conforme à l'une des revendications 1 à 5, **caractérisé en ce que** l'agent contient en outre des tensioactifs, en particulier des alkyl-sulfates ou des alkyl-sulfonates.
